(19) Europäisches Patentamt
European Patent Office
Office européen des brevets

(11) Veröffentlichungsnummer : **0 224 056 B1**

# (12) EUROPÄISCHE PATENTSCHRIFT

(45) Veröffentlichungstag der Patentschrift : **23.01.91 Patentblatt 91/04**

(51) Int. Cl.[5] : **C07D 249/08,** A01N 43/653

(21) Anmeldenummer : **86114929.2**

(22) Anmeldetag : **27.10.86**

## (54) Triazolylglykolether, Fungizide und Bioregulatoren.

Die Akte enthält technische Angaben, die nach dem Eingang der Anmeldung eingereicht wurden und die nicht in dieser Patentschrift enthalten sind.

(30) Priorität : **21.11.85 DE 3541156**

(43) Veröffentlichungstag der Anmeldung : **03.06.87 Patentblatt 87/23**

(45) Bekanntmachung des Hinweises auf die Patenterteilung : **23.01.91 Patentblatt 91/04**

(84) Benannte Vertragsstaaten : **AT BE CH DE ES FR GB IT LI NL SE**

(56) Entgegenhaltungen :
**EP-A- 0 056 087**
**EP-A- 0 092 674**

(73) Patentinhaber : **BASF Aktiengesellschaft**
**Carl-Bosch-Strasse 38**
**D-6700 Ludwigshafen (DE)**

(72) Erfinder : **Buschmann, Ernst, Dr.**
**Georg-Ludwig-Krebs-Strasse 10**
**D-6700 Ludwigshafen (DE)**
Erfinder : **Sproesser, Linhard, Dr.**
**Goethestrasse 4**
**D-6702 Bad Duerkheim (DE)**
Erfinder : **Zeeh, Bernd, Dr.**
**Queichstrasse 5**
**D-6703 Limburgerhof (DE)**
Erfinder : **Jung, Johann, Prof. Dr.**
**Hardenburgstrasse 19**
**D-6703 Limburgerhof (DE)**
Erfinder : **Rademacher, Wilhelm, Dr.**
**Austrasse 1**
**D-6703 Limburgerhof (DE)**
Erfinder : **Pommer, Ernst-Heinrich, Dr.**
**Berliner Platz 7**
**D-6703 Limburgerhof (DE)**
Erfinder : **Ammermann, Eberhard, Dr.**
**Sachsenstrasse 3**
**D-6700 Ludwigshafen (DE)**

**EP 0 224 056 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft neue Triazolylglykolether, fungizid und/oder pflanzenwachstumsregulierend wirkende Mittel, die diese Triazolylglykolether enthalten, sowie die Verwendung dieser Verbindungen bzw. Mittel zur Bekämpfung von Fungi bzw. zur Regulierung des Pflanzenwachstums.

Aus der DE-OS 30 47 726 ist bekannt, gewisse Triazolylether als Bioregulatoren zu verwenden. Die EP-A-92 674 beschreibt Triazolylglykolether mit einer Phenylglykoleinheit und einer tert.-Butylgruppe im Molekül. Die pflanzenwachstumsregulierende Wirkung dieser Substanzen, ihre Abbaubarkeit im Boden, artspezifische Anwendbarkeit und ihre Verträglichkeit gegenüber Kulturpflanzen wird jedoch nicht allen Anforderungen gerecht.

Es wurde nun gefunden, daß α-Triazolylglykolether I

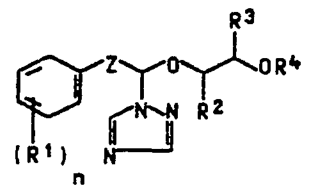

(I),

in der

$R^1$ für $C_1$-$C_4$-Alkyl, $C_1$-$C_4$-Alkoxy, Halogen, Phenyl,
$R^2$, $R^3$ für Wasserstoff, $C_1$-$C_4$ -Alkyl
$R^4$ für $C_1$-$C_4$-Alkyl,
Z für

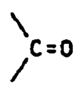 oder 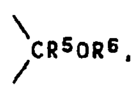

n für die Zahl 0, 1, 2 oder 3 steht,
wobei
$R^5$ Wasserstoff, $C_1$-$C_4$-Alkyl, Vinyl, Allyl, Prop-1-en-1-yl, Ethinyl, Prop-1-in-1-yl, But-1-in-1-yl und
$R^6$ Wasserstoff, $C_1$-$C_4$-Alkyl, Allyl, Crotyl, Benzyl, Halogenbenzyl, Alkylbenzyl oder Alkoxybenzyl bedeuten,
gute pflanzenwachstumsregulierende Wirkung haben, die der Wirkung der bekannten Triazolylether überlegen ist, und darüber hinaus fungizid wirken.

In der Formel I steht $R^1$ für einen Alkylrest wie Methyl, Ethyl, n-Propyl, iso-Propyl, n-Butyl, tert.-Butyl, iso-Butyl, oder einen entsprechenden Alkoxyrest mit 1 bis 4 C-Atomen, für Fluor, Brom, Chlor und eventuell Jod sowie für den Phenylrest.

$R^2$ und $R^3$ stehen unabhängig voneinander jeweils für Wasserstoff oder einen Alkylrest mit 1 bis 4 C-Atomen, nämlich Methyl, Ethyl, n-Propyl, iso-Propyl, n-Butyl, tert.-Butyl, iso-Butyl.

$R^4$ steht für einen Alkylrest wie Methyl, Ethyl, n-Propyl, iso-Propyl, n-Butyl, tert.-Butyl, iso-Butyl.

$R^5$ steht vorzugsweise für Wasserstoff oder für einen Alkylrest wie Methyl, Ethyl, n-Propyl, iso-Propyl, n-Butyl, iso-Butyl, ferner für Vinyl, Allyl, Prop-1-en-1-yl oder für Ethinyl, Prop-1-in-1-yl, But-1-in-1-yl.

$R^6$ steht vorzugsweise für einen Alkylrest wie Methyl, Ethyl, n-Propyl, iso-Propyl, n-Butyl, iso-Butyl, Allyl oder Crotyl, für den Benzyl-oder für Halogenbenzyl (3-Chlorbenzyl, 2-Chlorbenzyl, 2,4-Dichlorbenzyl, 4-Fluorbenzyl), Alkylbenzyl (4-Methylbenzyl, 4-tert.-Butylbenzyl), Alkoxybenzyl (4-Methoxybenzyl, 2-Methoxybenzyl, 4-Isopropoxybenzyl).

Die Triazolylglykolether besitzen ein oder mehrere Asymmetriezentren und bilden demnach unterschiedliche Diastereomere oder Enantiomere, die mit den üblichen Trenn-Methoden in reiner Form isoliert werden können. Als Mittel im Sinne der Erfindung sind sowohl die einheitlichen Enantiomere bzw. Diastereomere wie auch die bei der Synthese üblicherweise anfallenden Gemische geeignet.

Die Triazolylglykolether der Formel I lassen sich leicht herstellen, indem man ein entsprechendes Benzoylformaldehydacetal II

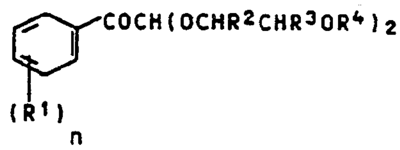

(II)

mit Acetylbromid und 1,2,4-Triazol zu einem Triazolylketon Ia

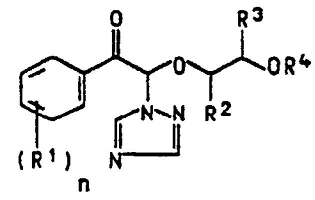

(Ia)

umsetzt. Das Keton kann mit üblichen Mitteln in die entsprechenden Carbinole Ib oder Ic und diese in die entsprechenden Ether Id bzw. Ie überführt werden :

Das Triazolylketon Ia kann entweder mit Reduktionsmittel wie $NaBH_4$, $LiAlH_4$, $H_2$/Katalysator übergeführt werden in ein Triazolylcarbinol Ib

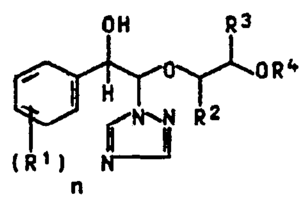

(Ib)

oder auch nach Grignard oder mittels einer Li-organischen Verbindung in ein Triazolylcarbinol Ic

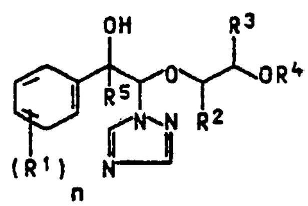

(Ic)

Alle diese Verbindungen sind erfindungsgemäße Wirkstoffe, ebenso die hieraus durch Verethern der Carbinole Ib oder Ic erhältlichen Ether Id bzw. Ie.

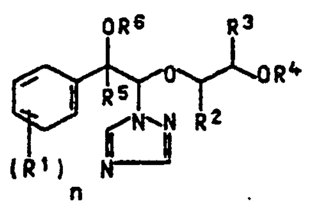

(Id→ $R^5$ = H→ $R^6$ ≠ H)
(Ie→ $R^5$ ≠ H→ $R^6$ ≠ H)

Die Benzoylformaldehydacetale der Formel (II) sind aus der japanischen Offenlegungsschrift J 57 210 076 (CA 96 (20) : 164 204 c) teilweise bekannt und lassen sich jedenfalls durch Umacetalisieren aus bekannten oder auf bekannte Weise herstellbaren Benzoylformaldehydacetalen gewinnen.

Die Herstellung der neuen Wirkstoffe wird an folgenden beispielhaften Umsetzungen erläutert :

Stufe A:

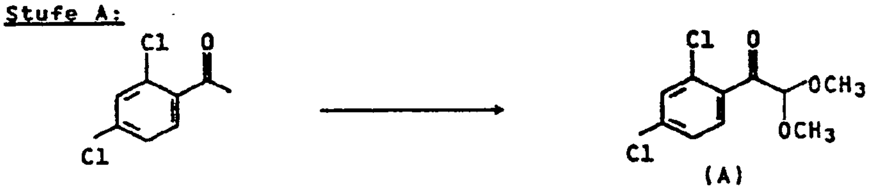

(A)

Zu einer Lösung von 567 g 2,4-Dichloracetophenon und 91 g HCl in 2 l Methanol werden bei Raumtemperatur 366 g Methylnitritgas zugeführt. Man rührt 5 Stunden nach, neutralisiert mit 30%igem Natriummethylat, saugt ab und nimmt das Filtrat mit Methylenchlorid auf, wäscht mit Wasser, trocknet über Na-Sulfat, engt ein und destilliert. Ausbeute : 535 g ω,ω-Dimethoxy-2,4-dichloracetophenon (A), Sdp. 132 - 6°C, 0,7 mbar.

Stufe B

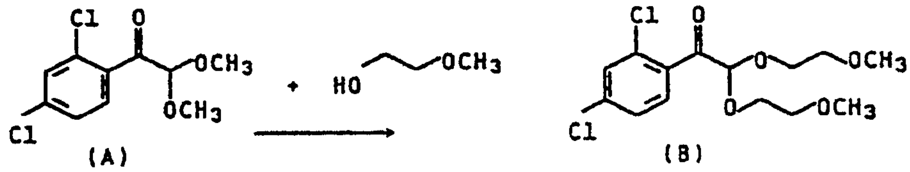

(A) (B)

Eine Lösung von 100 g (A) und 10 g HCl in 230 g Ethylenglykolmonomethylether wird 15 Stunden bei Raumtemperatur gerührt und anschließend an der Wasserstrahlpumpe langsam eingeengt. Falls sich ergibt (GC-Kontrolle), daß die Umsetzung unvollständig ist, wird erneut Ethylenglykolmonomethylether und HCl zugegeben und erneut eingeengt. Das Rohprodukt wird mit Methylenchlorid aufgenommen, mit wäßriger Na-Bicarbonat-Lösung gewaschen, über Na-Sulfat getrocknet und eingeengt. Destillation ergibt 60 g (B), Sdp. 175 - 180°C, 0,2 mbar.

Stufe C

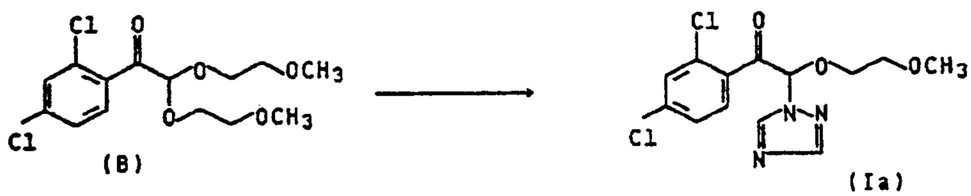

(B) (Ia)

Zu 60 g (B) werden bei unter 40°C 22 g Acetylbromid allmählich zugesetzt. Das erhaltene Zwischenprodukt wird ohne weitere Reinigung zu einer Lösung von 25 g 1,2,4-Triazol in 200 ml Tetrahydrofuran und 60 ml Dimethylformamid zugetropft. Die Temperatur soll dabei 30°C nicht übersteigen. Man rührt 13 Stunden bei Raumtemperatur, saugt ab, engt das Filtrat ein, nimmt mit 500 ml Methylenchlorid auf, wäscht mit Wasser, trocknet über Na-Sulfat und engt ein.

Zur etherischen Lösung des Rückstandes wird unter Eiskühlung 98%ige Salpetersäure zugetropft. Das dabei gebildete Nitrat, das einem Wirkstoff der Formel Ia entspricht, wird im Vakuum getrocknet – 35 g Ausbeute, Fp. 113°C (Diese Verbindung ist in der nachstehenden Tabelle als Beispiel Nr. 18 aufgeführt.).

Das Nitrat wird in Methanol gelöst. Man stellt mit wäßrigem Ammoniak alkalisch, engt ein und nimmt mit Methylenchlorid auf. Die Lösung wird mit Wasser gewaschen, über Na-Sulfat getrocknet und eingeengt. Destillation des Rückstandes ergibt 20 g (Sdp. 183 - 187°C /0,5 mbar) eines Wirkstoffs der als Beispiel Nr. 17 in der Tabelle aufgeführt ist.

Stufe D

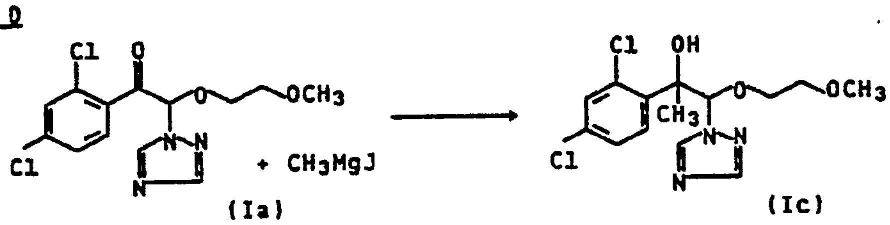


(Ia) (Ic)

Zu einer Suspension von 0,35 Mol $CH_3MgJ$ in 500 ml Diethylether werden 57 g Ia in 200 ml Ether zugetropft. Man rührt 2 Stunden unter Rückfluß, hydrolysiert mit gesättigter wäßriger Ammonchloridlösung und extrahiert mit Diethylether. Der Extrakt wird mit wäßriger Na-Thiosulfatlösung und mit Wasser gewaschen, über Na-Sulfat getrocknet und auf 200 ml eingeengt. Zur eisgekühlten etherischen Lösung wird 98%ige Salpetersäure zugetropft. Das dabei ausfallende Nitrat wird aus Essigester umkristallisiert. Man erhält 16 g (Schmelzpunkt 133 - 134°C) des Wirkstoffs, der als Beispiel Nr. 20 in der Tabelle aufgeführt ist.

Das Nitrat läßt sich wie beschrieben zur freien Base umsetzen (Beispiel Nr. 21).

Stufe E

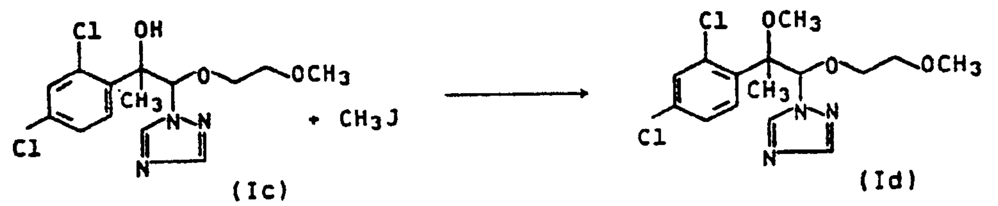

(Ic) (Id)

Zur Lösung von 29 g (Ic) und 14,5 g $CH_3J$ in 390 ml Diethylether und 110 ml Dimethylsulfoxid werden 3 g Natriumhydrid als 80%ige Paraffinsuspension portionsweise zugegeben. Nach Ende der Wasserstoffentwicklung wird 30 Minuten am Rückfluß erwärmt. Man versetzt langsam mit 1,2 l Wasser und extrahiert wiederum mit Ether. Die organische Phase wird mit wäßriger Na-Thiosulfat-Lösung und mit Wasser gewaschen, über Na-Sulfat getrocknet und eingeengt. Umkristallisation des Rohproduktes aus Diisopropylether ergibt 19 g des Wirkstoffs (Fp. 122 - 123°C), der als Beispiel Nr. 24 in der Tabelle aufgeführt ist.

In der nachstehenden Tabelle sind weitere erfindungsgemäße Verbindungen aufgeführt; soweit physikalische Eigenschaften dazu angegeben sind, wurden sie hergestellt und auf ihre Wirkung untersucht. Die anderen Verbindungen können unter sinngemäßer Abwandlung der vorstehenden Hinweise hergestellt werden; sie lassen aufgrund ihrer strukturellen Verwandtschaft eine gleichartige Wirkung erwarten.

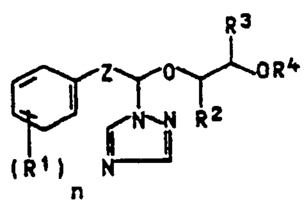

(HX)

| Nr. | $(R^1)_n$ | Z | $R^2$ | $R^3$ | $R^4$ | Fp °C (·HX) | Sdp. °C/mbar |
|---|---|---|---|---|---|---|---|
| 1 | H | C=O | H | H | $CH_3$ | | |
| 2 | H | CHOH | H | H | $CH_3$ | | |
| 3 | H | $CCH_3OH$ | H | H | $CH_3$ | | |
| 5 | 4-$CH_3$ | C=O | H | H | $CH_3$ | | |
| 6 | 4-$CH_3$ | $CCH_3OH$ | H | H | $CH_3$ | | |
| 7 | 4-$OCH_3$ | C=O | H | H | $CH_3$ | | |
| 8 | 4-$OCH_3$ | $CCH_3OH$ | H | H | $CH_3$ | | |
| 9 | 2-Cl | C=O | H | H | $CH_3$ | | |
| 10 | 2-Cl | $CCH_3OH$ | H | H | $CH_3$ | | |
| 11 | 4-$C_6H_5$ | C=O | H | H | $CH_3$ | | |
| 12 | 4-$C_6H_5$ | $CCH_3OH$ | H | H | $CH_3$ | | |
| 13 | 4-Cl | C=O | H | H | $CH_3$ | | |
| 14 | 4-Cl | $CCH_3OH$ | H | H | $CH_3$ | | |
| 15 | 4-tBn | C=O | H | H | $CH_3$ | | |
| 16 | 4-tBn | $CCH_3OH$ | H | H | $CH_3$ | | |
| 17 | 2,4-$Cl_2$ | C=O | H | H | $CH_3$ | | 183-7°/0,3 |
| 18 | 2,4-$Cl_2$ | C=O | H | H | $CH_3$ | 113° .$HNO_3$ | |
| 19 | 2,4-$Cl_2$ | CHOH | H | H | $CH_3$ | | |
| 20 | 2,4-$Cl_2$ | $CCH_3OH$ | H | H | $CH_3$ | 133-4° .$HNO_3$ | |
| 21 | 2,4-$Cl_2$ | $CCH_3OH$ | H | H | $CH_3$ | | |
| 22 | 2,4-$Cl_2$ | $CCH=CH_2OH$ | H | H | $CH_3$ | | |
| 23 | 2,4-$Cl_2$ | $CC_2H_5OH$ | H | H | $CH_3$ | | |
| 24 | 2,4-$Cl_2$ | $CCH_3OCH_3$ | H | H | $CH_3$ | 122-3° | |

| Nr. | $(R^1)_n$ | Z | $R^2$ | $R^3$ | $R^4$ | Fp °C (·HX) | Sdp. °C/mbar |
|---|---|---|---|---|---|---|---|
| 25 | 2,4-$Cl_2$ | $COCH_2CH=CH_2$<br>$CH_3$ | H | H | $CH_3$ | | 170-4°/03 |
| 26 | 2,4-$Cl_2$ | $COCH_2C_6H_5$<br>$CH_3$ | H | H | $CH_3$ | | |
| 27 | 2,4-$Cl_2$ | C=O | H | $CH_3$ | $CH_3$ | | |
| 28 | 2,4-$Cl_2$ | $CCH_3OH$ | H | $CH_3$ | $CH_3$ | | |
| 29 | 2,4-$Cl_2$ | C=O | H | H | $C_2H_5$ | 101° .$HNO_3$ | |
| 30 | 2,4-$Cl_2$ | CHOH | H | H | $C_2H_5$ | | |
| 31 | 2,4-$Cl_2$ | $CCH_3OH$ | H | H | $C_2H_5$ | 148° .$HNO_3$ | |
| 32 | 2,4-$Cl_2$ | $CCH_3OH$ | H | H | $C_2H_5$ | 80° | |
| 33 | 2,4-$Cl_2$ | $CCH=CH_2OH$ | H | H | $C_2H_5$ | | |
| 34 | 2,4-$Cl_2$ | $COCH_2CHCH_2$<br>$CH_3$ | H | H | $C_2H_5$ | | |
| 35 | 2,4-$Cl_2$ | $CC_2H_5OH$ | H | H | $C_2H_5$ | | |
| 36 | 2,4-$Cl_2$ | $CCH_3OCH_3$ | H | H | $C_2H_5$ | | |
| 37 | 2,4-$Cl_2$ | $CCH_3OH$ | $CH_3$ | $CH_3$ | $CH_3$ | | |
| 38 | 2,4-$Cl_2$ | C=O | H | H | iso-Propyl | | |
| 39 | 2,4-$Cl_2$ | $CCH_3OH$ | H | H | iso-Propyl | | |

Beispiele für die Anwendung als Wachstumsregulatoren

(A)

An Sommergerste und Ackerbohnen wurden Vegetationsversuche in Mitscherlichgefäßen durchgeführt. Der Pflanzenaufwuchs erfolgte auf einem lehmigen Sandboden, der durch eine einheitliche Grunddüngung ausreichend mit Nährstoffen versorgt war. Die Applikation der Wirkstoffe wurde bei definierten Pflanzenhöhen durch Spritzen über das Blatt vorgenommen. Zu Versuchsende wurde die Wuchshöhe der Pflanzen gemessen. Als Vergleich wurde die Verbindung des Beispiels Nr. 11 der DE-OS 30 47 726 gewählt.

Es zeigt sich, daß die erfindungsgemäße Verbindung Nr. 31 eine überlegene wuchshemmende Wirkung besitzt, d.h. es tritt deutlich gedrungener Wuchs bei gesundem Aussehen der Testpflanzen auf, wobei eine geringere Aufwandmenge als beim Vergleich benötigt wird.

(B)

Zur Bestimmung der wachstumsregulierenden Eigenschaft der Prüfsubstanzen wurden Testpflanzen auf ausreichend mit Nährstoffen versorgtem Kultursubstrat in Kunststoffgefäßen von ca. 12,5 cm Durchmesser angezogen.

Im Vorauflaufverfahren wurden die Testsubstanzen in wäßriger Aufbereitung am Tage der Einsaat auf das Saatbett gegossen. Im Nachauflaufverfahren wurden die zu prüfenden Substanzen in wäßriger Aufbereitung auf die Pflanzen gesprüht.

Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Als Vergleichssubstanz diente die gleiche Substanz wie beim vorstehenden Versuch.

In diesen Versuchen, die u.a. an Sommerweizen, Sommergerste, Reis, Sonnenblumen, Sommerraps

und Soja vorgenommen wurden, zeigten z.B. die Wirkstoffe 20, 21, 29 und 31 eine besonders günstige Wirkung auf das Längenwachstum.

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Beispiele für die Anwendung als Fungizide

(C) Wirksamkeit gegen Weizenmehltau ; vorbeugende Spritzung

Blätter von in Töpfen gewachsenen Weizenkeimlingen der Sorte "Frühgold" werden mit einer wäßrigen Spritzbrühe besprüht, die aus einem 80% Wirkstoff und 20% Emulgiermittel enthaltenden Konzentrat durch Verdünnen erhalten worden war, und 24 Stunden nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80% relativer Luftfeuchtigkeit aufgestellt. Nach 7 Tagen wird das Ausmaß der Mehltauentwicklung ermittelt. Die Bewertung erfolgt in der Weise, daß der Pilzbefall mit 0 bis 5 und die Blattschädigung mit A, B, C oder T (Totalschaden) klassifiziert werden. Bei diesem Versuch erzielen die Wirkstoffe 18, 20, 21, 24, 31, 32 schon mit 0,06%iger Spritzbrühe fast durchweg vollständige Hemmung des Befalls, während der unbehandelte Vergleich Totalbefall zeigt.

(D) Wirksamkeit gegen Gurkenmehltau ; Behandlung nach Sporeneinwirkung

Blätter von in Töpfen gewachsenen Gurkenkeimlingen der Sorte "Chinesische Schlange" werden im Zweiblattstadium mit einer Sporensuspension des Gurkenmehltaus besprüht. Nach etwa 20 Stunden werden die Versuchspflanzen mit einer wäßrigen Spritzbrühe, die aus einem 80% Wirkstoff und 20% Emulgiermittel enthaltenden Konzentrat durch Verdünnen erhalten worden war, bis zur Tropfnässe besprüht. Nach dem Antrocknen des Spritzbelages werden sie anschließend im Gewächshaus bei Temperaturen zwischen 20 und 22°C und 70 bis 80% relativer Luftfeuchtigkeit aufgestellt. Zur Beurteilung der Wirksamkeit der neuen Stoffe wird das Ausmaß der Pilzentwicklung nach 21 Tagen ermittelt.

In diesem Versuch, bei dem wieder der Befall mit 0 bis 5 bewertet wird, verhindert eine Anwendungskonzentration von 0,025% der Wirkstoffe 18, 20, 31, 32 in praktisch allen Fällen das Auftreten von Schadbildern, während der unbehandelte Vergleich Totalbefall zeigt.

(E) Wirksamkeit gegen Weizenbraunrost ; Behandlung nach Infektion

Blätter von in Töpfen gewachsenen Weizensämlingen der Sorte "Frühgold" werden mit Sporen des Braunrostes (Puccinia recondita) bestäubt. Danach werden die Töpfe für 24 Stunden bei 20 bis 22°C in eine Kammer mit hoher Luftfeuchtigkeit (90 bis 95%) gestellt. Während dieser Zeit keimen die Sporen aus und die Keimschläuche dringen in das Blattgewebe ein. Dies führt ohne Behandlung mit Fungiziden zum Verlust der Pflanze ; die Infektion wird durch einen Kontrollversuch bestätigt.

Die infizierten Pflanzen werden anschließend mit einer wäßrigen Spritzbrühe, die aus einem 80% Wirkstoff und 20% Emulgiermittel enthaltenden Konzentrat durch Verdünnen erhalten worden war, tropfnaß gespritzt. Nach dem Antrocknen des Spritzbelages werden die Versuchspflanzen im Gewächshaus bei Temperaturen zwischen 20 und 22°C und 65 bis 70% relativer Luftfeuchte aufgestellt. Nach 8 Tagen wird das Ausmaß der Rostpilzentwicklung auf den Blättern ermittelt.

Die Bewertung geschieht, wie vorstehend beschrieben ; dabei verhindern die Wirkstoffe Nr. 20, 21, 24, 31 und 32 schon bei 0,006%iger Konzentration in der Spritzbrühe den sichtbaren Befall wesentlich, während bei 0,025%iger Anwendung praktisch vollständiger Schutz erreicht wird.

(F) Wirksamkeit gegen Pyrenophora teres ; vorbeugende Behandlung

Gerstenkeimlinge der Sorte "Asse" werden im Zweiblattstadium mit wäßrigen Suspensionen, die 80% Wirkstoff und 20% Emulgator in der Trockensubstanz enthalten, tropfnaß gespritzt. Nach 24 Stunden werden die Pflanzen mit einer Sporensuspension des Pilzes Pyrenophora teres inoculiert und für 48 Stunden in eine Klimakammer mit hoher Luftfeuchtigkeit bei 18°C gestellt. Anschließend werden die Pflanzen im Gewächshaus bei 20-22°C und 70% relativer Luftfeuchtigkeit für weitere 5 Tage kultiviert. Dann wird das Ausmaß der Symptomentwicklung ermittelt und wie vorstehend angegeben bewertet.

Die Verbindungen 20, 21 und 31 hatten in diesen Versuchen schon in 0,05%iger Zubereitung gute bis

ausgezeichnete Wirkung, während die unbehandelten Kulturpflanzen verlorengingen.

Die neuen Verbindungen zeichnen sich, wie die vorstehenden Versuche zeigen, auch durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind z.B. geeignet zur Bekämpfung folgender Pflanzenkrankheiten :

Erysiphe graminis (echter Mehltau) in Getreide,
Erysiphe cichoracearum an Kürbisgewächsen,
Podosphaera leucotricha an Äpfeln,
Uncinula necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Hemileia vastatrix an Kaffee,
Alternaria solani an Kartoffeln, Tomaten,
Plasmopara viticola an Reben sowie Fusarium- und Verticillium-Arten an verschiedenen Pflanzen.

Wirkung der erfindungsgemäßen Stoffe als Wachstumsregulatoren

## Beispiel 1

Gewächshausversuche

Zur Bestimmung der wachstumsregulierenden Eigenschaft der Wirkstoffe wurden Testpflanzen auf ausreichend mit Nährstoffen versorgtem Kultursubstrat in Kunststoffgefäßen (ca. 12,5 cm Durchmesser) angezogen.

Im Nachauflaufverfahren wurden die Wirkstoffe in wäßriger Aufbereitung auf die Pflanzen gesprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Als Vergleichssubstanz diente der Wirkstoff des Beispiels 11 der DE-OS-30 47 726 (A).

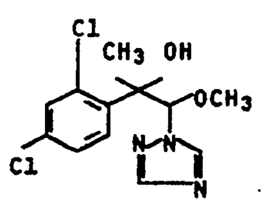

(A)

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Die Einzeldaten sind den folgenden Tabellen zu entnehmen.

Tabelle 1

Sommerweizen, Sorte "Kolibri"

Nachauflauf-Blattbehandlung

| Nr. d. chem. Beispiele | Konz. mg WS/Gef. | Wuchshöhen rel. |
|---|---|---|
| unbehandelt | - | 100 |
| A | 6 | 93,7 |
| 20 | 6 | 88,5 |
| 21 | 6 | 87,7 |
| 31 | 6 | 82,3 |
| 32 | 6 | 85,9 |

Tabelle 2

Sommergerste, Sorte "Aramir"

Nachauflauf-Blattbehandlung

| Nr. d. chem. Beispiele | Konz. mg WS/Gef. | Wuchshöhen rel. |
|---|---|---|
| unbehandelt | - | 100 |
| A | 6 | 94,0 |
| 20 | 6 | 93,6 |
| 21 | 6 | 86,4 |
| 31 | 6 | 75,3 |
| 32 | 6 | 89,1 |

Tabelle 3

Reis, Sorte "Nihonbare"

Nachauflauf-Bodenbehandlung

| Nr. d. chem. Beispiele | Konz. mg WS/Gef. | Wuchshöhen rel. |
|---|---|---|
| unbehandelt | - | 100 |
| A | 1,5 | 99,8 |
| | 6 | 99,8 |
| 20 | 1,5 | 98,8 |
| | 6 | 88,9 |
| 31 | 1,5 | 93,8 |
| | 6 | 82,3 |
| 32 | 1,5 | 91,2 |
| | 6 | 84,3 |

Tabelle 4

Sonnenblumen, "Sorex"

Nachauflauf-Blattbehandlung

| Nr. d. chem. Beispiele | Konz. mg WS/Gef. | Wuchshöhen rel. |
|---|---|---|
| unbehandelt | - | 100 |
| A | 6 | 96,8 |
| 20 | 6 | 90,2 |
| 21 | 6 | 84,4 |
| 29 | 6 | 79,5 |
| 31 | 6 | 78,4 |

Tabelle 5

Sommerraps, "Petranova"

Nachauflauf-Blattbehandlung

| Nr. d. chem. Beispiele | Konz. mg WS/Gef. | Wuchshöhen rel. |
|---|---|---|
| unbehandelt | - | 100 |
| A | 6 | 92,4 |
| 20 | 6 | 85,0 |
| 21 | 6 | 69,4 |
| 29 | 6 | 79,6 |
| 31 | 6 | 72,6 |
| 32 | 6 | 68,8 |

Tabelle 6

Soja "Maple Arrow"

Nachauflauf-Blattbehandlung

| Nr. d. chem. Beispiele | Konz. mg WS/Gef. | Wuchshöhen rel. |
|---|---|---|
| unbehandelt | - | 100 |
| A | 0,5 | 92,5 |
| | 1,5 | 92,5 |
| 20 | 0,5 | 91,7 |
| | 1,5 | 86,2 |
| 31 | 0,5 | 84,4 |
| | 1,5 | 73,4 |

Beispiel 2

Vegetationsversuche

An Sommergerste und Ackerbohnen wurden Vegetationsversuche in Mitscherlichgefäßen durchgeführt. Der Pflanzenaufwuchs erfolgte auf einem lehmigen Sandboden, der durch eine einheitliche Grunddüngung ausreichend mit Nährstoffen versorgt war. Die Applikation der Wirkstoffe wurde bei definierten Pflanzenhöhen durch Spritzen über das Blatt vorgenommen. Zu Versuchsende wurde die Wuchshöhe der Pflanzen gemessen. Als Vergleichssubstanz diente der Wirkstoff des Beispiels Nr. 11 der DE-OS 30 47 726 (A).

Es konnte gezeigt werden, daß die erfindungsgemäße Verbindung Nr. 31 eine überlegene wuchshemmende Wirkung besaß.

**Vegetationsversuch bei Sommergerste**

| Nr. d. chem. Beispiele | Aufwandmenge mg/Gef. | Wuchshöhen cm |
|---|---|---|
| unbehandelt | - | 61,0 |
| A | 10 | 57,5 |
| | 20 | 52,0 |
| 31 | 10 | 54,8 |
| | 20 | 51,0 |

**Vegetationsversuch bei Ackerbohnen**

| Nr. d. chem. Beispiele | Aufwandmenge mg/Gef. | Wuchshöhen cm |
|---|---|---|
| unbehandelt | - | 83,3 |
| A | 5 | 81.0 |
| | 10 | 80.0 |
| 31 | 5 | 73,5 |
| | 10 | 70,0 |

## Ansprüche

1. α-Triazolylglykolether der Formel I

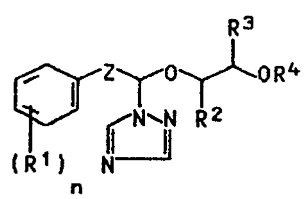

(I),

in der
$R^1$ für $C_1$-$C_4$-Alkyl, $C_1$-$C_4$-Alkoxy, Halogen, Phenyl,
$R^2$, $R^3$ für Wasserstoff, $C_1$-$C_4$-Alkyl,
$R^4$ für $C_1$-$C_4$-Alkyl,
Z für

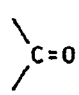 oder 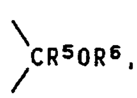

n für die Zahl 0, 1, 2 oder 3 steht,
wobei
$R^5$ Wasserstoff, $C_1$-$C_4$-Alkyl, Vinyl, Allyl, Prop-1-en-1-yl, Ethinyl, Prop-1-in-1-yl, But-1-in-1-yl und

$R^6$ Wasserstoff, $C_1$-$C_4$-Alkyl, Allyl, Crotyl, Benzyl, Halogenbenzyl, Alkylbenzyl oder Alkoxybenzyl bedeuten.

2. Verfahren zur Herstellung eines Triazolylglykolethers I gemäß Anspruch 1, dadurch gekennzeichnet, daß man ein entsprechendes Benzoylformaldehydacetal II

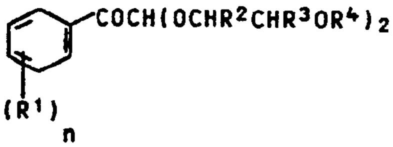

(II)

mit 1,2,4-Triazol und Acetylbromid zu einem entsprechenden Triazolylketon Ia

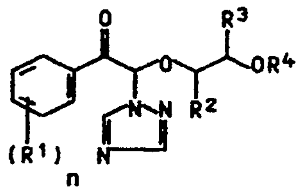

(Ia)

und dieses, falls Z = $CR_5OH$ sein soll, zum entsprechenden Carbinol Ib oder Ic

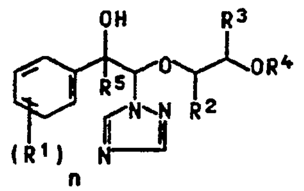

(Ib→ $R_5$ = H)
(Ic→ $R_5 \neq$ H)

sowie, falls Z = $CR_5OR_6$ sein soll, das Carbinol Ib oder Ic zum entsprechenden Ether Id bzw. Ie

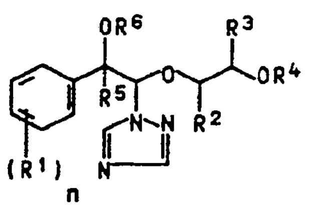

(Id→ $R^5$ = H→ $R^6 \neq$ H)
(Ie→ $R^5 \neq$ H→ $R^6 \neq$ H)

umsetzt.

3. Fungizides Mittel, enthaltend einen Triazolylglykolether gemäß Anspruch 1 und wenigstens einen Hilfsstoff.

4. Mittel zur Regulierung des Pflanzenwachstums, enthaltend einen Triazolylglykolether gemäß Anspruch 1 und wenigstens einen Hilfsstoff.

5. Verfahren zur Bekämpfung von Pilzen, dadurch gekennzeichnet, daß man die Pilze oder die von Pilzbefall bedrohten Materialien, Pflanzen, Böden oder Saatgüter behandelt mit einer fungizid wirksamen Menge eines Triazolylglykolethers gemäß Anspruch 1.

6. Verfahren zur Regulierung des Pflanzenwachstums, dadurch gekennzeichnet, daß man eine Verbindung gemäß Anspruch 1 auf die Pflanzen oder deren Vermehrungsgut einwirken läßt.

## Claims

1. An α-triazolyl glycol ether of the formula I

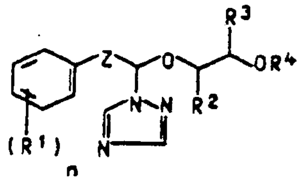

(I)

where
R$^1$ is $C_1$-$C_4$-alkyl, $C_1$-$C_4$-alkoxy, halogen or phenyl,
R$^2$ and R$^3$ are each hydrogen or $C_1$-$C_4$-alkyl,
R$^4$ is $C_1$-$C_4$-alkyl,
Z is

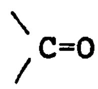 or 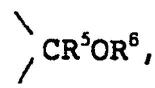,

n is 0, 1, 2 or 3,
R$^5$ is hydrogen, $C_1$-$C_4$-alkyl, vinyl, allyl, prop-1-en-1-yl, ethynyl, prop-1-yn-1-yl or but-1-yn-1-yl, and
R$^6$ is hydrogen, $C_1$-$C_4$-alkyl, allyl, crotyl, benzyl, halobenzyl, alkylbenzyl or alkoxybenzyl.

2. A process for the preparation of a triazolyl glycol ether I as claimed in claim 1, wherein an appropriate benzoylformaldehyde acetal II

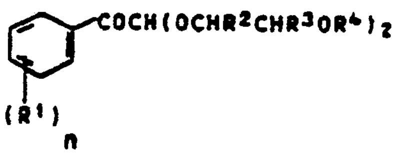

(II)

is reacted with 1,2,4-triazole and acetyl bromide to give a corresponding triazolyl ketone Ia

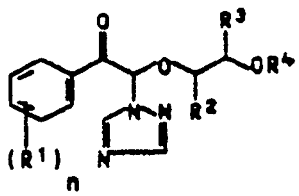

(Ia)

and, where Z is intended to be $CR_5OH$, the triazolyl ketone Ia is converted to the corresponding carbinol Ib or Ic

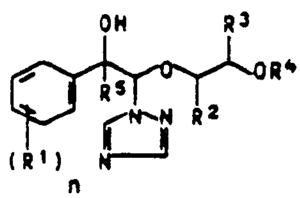

(Ib→ $R_5$ = H)
(Ic→ $R_5$ ≠ H)

and, where Z is intended to be $CR_5OR_6$, the carbinol Ib or Ic is converted to the corresponding ether Id or Ie

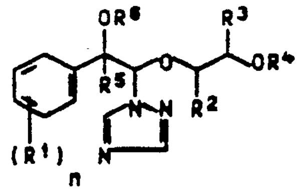

(Id→ R5 = H→ R6 ≠ H)
(Ie→ R5 ≠ H→ R6 ≠ H)

3. A fungicide containing a triazolyl glycol ether as claimed in claim 1 and one or more assistants.

4. A plant growth regulator containing a triazolyl glycol ether as claimed in claim 1 and one or more assistants.

5. A method of controlling fungi, wherein the fungi or the materials, plants, soils or seeds which are threatened with fungal attack are treated with a fungicidally effective amount of a triazolyl glycol ether as claimed in claim 1.

6. A method for regulating plant growth, wherein a compound as claimed in claim 1 is allowed to act on the plants or their propagation stock.

**Revendications**

1. α-triazolylglycoléther de la formule I

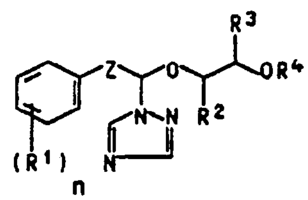

(I),

dans laquelle
$R^1$ est mis pour alkyle en $C_1$-$C_4$, alcoxy en $C_1$-$C_4$ halogène, phényle,
$R^2$, $R^3$, pour hydrogène, alkyle en $C_1$-$C_4$
$R^4$, pour alkyle en $C_1$-$C_4$
Z, pour

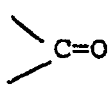

ou

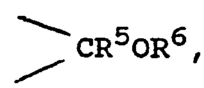

n, pour le nombre 0, 1, 2 ou 3
$R^5$ représentant hydrogène, alkyle en $C_1$-$C_4$, vinyle, allyle, prop-1-en-1-yle, éthinyle, prop-1-in-1-yle, but-1-in-1-yle et hydrogène, alkyle en $C_1$-$C_4$, allyle, crotyle, benzyle, halogènobenzyle, alkylbenzyl ou alcoxybenzyle.

2. Procédé de préparation d'un triazolylglycoléther I selon la revendication 1, caractérisé par le fait qu'on fait réagir un benzoylformaldéhydacétal II

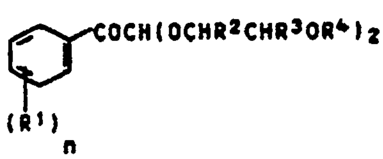

(II)

avec du 1,2,4-triazole et du bromure d'acétyle pour obtenir une triazolylcétone correspondante la

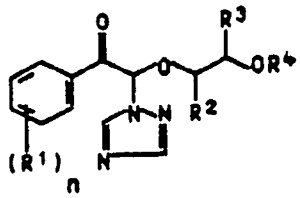

(Ia)

et l'on transforme celle-ci, lorsque Z doit être $CR_5OH$, en le carbinol $I_b$ ou $I_c$ correspondant

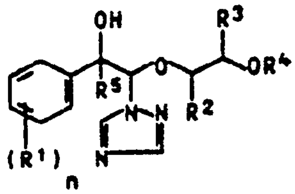

(Ib→ $R_5$ = H)
(Ic→ $R_5$ ≠ H)

et dans le cas, Z doit être $CR_5OR_6$, on transforme le carbinol $I_b$ ou $I_c$ en éther $I_d$ ou $I_e$ correspondant

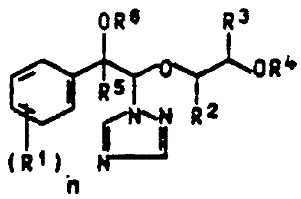

(Id→ $R^5$ = H→ $R^6$ ≠ H)
(Ie→ $R^5$ ≠ H→ $R^6$ ≠ H)

3. Agent fongicide, contenant un triazolylglycoléther, selon la revendication 1 et au moins un auxiliaire.

4. Agent de régularisation de la croissance des plantes contenant un triazolylglycoléther, selon la revendication 1 et au moins un auxiliaire.

5. Procédé de lutte contre les champignons, caractérisé par le fait que l'on traite les champignons ou les matériaux, plantes, sols ou semences, menacés d'une attaque par les champignons, avec une quantité efficace du point de vue fongicide d'un triazolylglycoléther selon la revendication 1.

6. Procédé de régularisation de la croissance des plantes caractérisé par le fait qu'on fait agir un composé selon la revendication 1 sur les plantes ou leur domaine de multiplication.